# EUROPEAN PATENT SPECIFICATION

(11) **EP 0 340 378 B1**
(45) Date of publication and mention of the grant of the patent: **21.07.1993**
(21) Application number: 88870078.8
(22) Date of filing: 05.05.1988
(51) Int. Cl.: C12N 11/04, C12N 11/10

(54) **Method for microbial immobilization**
Verfahren zur Immobilisierung von Mikroben
Procédé d'immobilisation de microbes

(43) Date of publication of application: 08.11.1989
(73) Proprietor: Monsanto Company, St. Louis Missouri 63167-7020 (US)
(72) Inventor: Nolan, Carol Louise, Chesterfield Missouri 63017 (US)
(74) Representative: Ernst, Hubert

(56) References cited:
- EP-A- 0 012 552
- EP-A- 0 130 689
- FR-A- 2 158 330
- GB-A- 2 116 997
- GB-A- 2 145 992
- GB-A- 2 149 816
- PATENT ABSTRACTS OF JAPAN, vol. 8, no. 225 (C-247)[1662], 16th October 1984
- PROCESS BIOCHEMISTRY, August 1972, pages 9-12; D. DINELLI: "Fibre-entrapped enzymes"
- CHEMICAL ABSTRACTS, vol. 109, no. 3, 18th July 1988, page 648, abstract no. 23249w, Columbus, Ohio, US; H. GRASDALEN et al.: "Gelation of gellan gum", & CARBOHYDR. POLYM. 1987, 7(5), 371-93
- CHEMICAL ABSTRACTS, vol. 107, no. 10, 7th September 1987, page 114, abstract no. 79869z, Columbus, Ohio, US; G.R. CHILVERS et al.: "Coacervation of gelatin-gellan gum mixtures and their use in microencapsulation", & CARBOHYDR. POLYM. 1987, 7(2), 111-20

## Description

The present invention relates to immobilized microbial cells which are catalytically-active and to a method for preparing same in which the immobilization is effected by means of entrapping whole microbial cells or catalytically active biological material with a composition essentially comprising a polysaccharide containing glucuronic acid, rhamnose and glucose. Various techniques have been proposed for immobilizing microbial cells and enzymes on water-insoluble supports. These methods have included covalent chemical linkage to a support using functional groups of the enzyme that are not essential for enzymatic activity; entrapment of the microbial cell or enzyme within a hydrophilic gel lattice which retains the catalytically-active material while permitting substrate and product to pass through; ionic binding or physical absorption on hydrophilic ion exchange resins or on charcoal or glass beads and aggregating the enzymes by cross-linking with bifunctional compounds.

Entrapment methods of immobilization are most versatile. For example, U.S. Patent 3,791,926 discloses methods of entrapping microbial cells and enzymes within polymer matrices. U.S. Patent 3,957,580 discloses a method of entrapping microbial cells within polymer systems in which the immobilized cells are further cross-linked to the polymer matrix using poly-functional reagents such as glutaraldehyde.

However, synthetic polymer systems of the types described above have several drawbacks. The preparation of immobilized cells may involve the use of toxic materials such as monomers, initiators and cross-linkers. The support matrix can deform or compress upon use in a commercial-scale bioreactor system.

Accordingly, it is the overall object of the present invention to provide a method of immobilizing, catalytically-active microbial cells which maintains enzymatic activity.

It is another object of the present invention to provide a method of immobilizing catalytically-active microbial cells which results in compositions exhibiting good mechanical strength.

It is still another object of the present invention to provide an immobilization method which results in composition exhibiting kinetics comparable to soluble enzymes.

It is yet another object of the present invention to provide an immobilization method which uses an encapsulation polymer which is easy to use and inexpensive.

These and other objects and advantages will be evident from the following description and illustrative examples.

### DESCRIPTION OF THE INVENTION

The present invention provides an improved method for microbial cell immobilization in which the cells are encapsulated in a composition essentially comprising a polysaccharide containing glucouronic acid, rhamnose and glucose. More particularly the invention employs the use of a composition known as gellan gum. For purposes of the present invention by "gellan gum" is meant the deacetylated heteropolysaccharide S-60 as described and claimed in U.S. 4,326,052 issued April 20, 1982 to K. S. Kang et al. The immobilized cell preparations which result from the present method show surprising advantages over previously disclosed encapsulation methods: mechanical strength, enzymatic stability, easy/inexpensive preparation, and kinetics comparable to the soluble enzyme.

The preferred method of obtaining stable, immobilized, catalytically-active substances is by hardening droplets formed from a mixture of microorganisms, gellan gum, and water with a solution of MgSO₄ or other salt containing monovalent or divalent cations. Although various other shapes are possible, spherical beads are preferred since this shape affords the greatest surface area per unit volume. The present method allows significant flexibility in determining the mechanical strength of the encapsulated material. In the present method, 1.2 to 1.8 wt% gellan gum is dissolved in deionized water, heated to at least 80°C and cooled to about 25-55°C, and mixed with a paste of microorganisms weighing not more than 50% of the weight of the gellan gum solution, preferably less than about 25%. The gellan gum/cell mixture is shaped in the form of beads by conventional methods known in the art and then are preferably contacted with a 0.1-0.4M solution of magnesium sulfate (MgSO₄) or another divalent cationic salt at room temperature. The resulting beads are allowed to harden for about 10 minutes and are then filtered from the MgSO₄ solution. If desired, the magnesium sulfate solution may be reused. If monovalent cationic salt solutions are used to harden the beads, higher concentrations, about 0.5-2.0M salt, are required. The preferred limits on the allowable concentrations of gellan gum, cell paste, and MgSO₄ solution are narrow since conditions outside of these ranges yield beads that are either too compressible or a mixture that is too viscous to be easily shaped into beads and prone to premature hardening.

Beads may be easily formed by forcing the gellan gum/cell mixture through a small bore pipette or syringe. The size of the bead may be controlled by a concentric flow of air around the pipette or syringe. Other methods of bead making may be employed such as those described by C. D. Scott, 1985 Int′l Enzyme Conference; U. Matulovic et al., Biotechnology Letters Vol. 8, No. 7, p 485-490 (1986); T. Rehg et al., Biotechnology Letters, Vol. 8, No. 2, pp 111-114 (1986); and A. C. Hulst et al., Biotech Bioengr., Vol. 17, pp 870-876 (1985).

It has been found that unlike other entrapment methods, the use of gellan gum is very sensitive to the conditions selected. One reason for this sensitivity is the small amount of gellan gum used in the present method. About one-half to one-third as much gellan gum is used in comparison to carrageenan and the resulting beads are mechanically stronger.

Small variations in the concentration of gellan gum provide flexibility in the type of bead which is formed. The gellan gum concentration should be sufficient to produce beads with adequate strength for the anticipated use but low enough to prevent premature hardening of the gellan gum. Preferably, initial concentrations (before the addition of cell paste) of gellan gum should not be below 1.2% or the beads will be easily deformed and, in many cases, unsuitable for column reactors. Initial concentrations of gellan gum above 1.8 wt% may create problems in dispensing the gellan gum/cell mixture into droplets without premature hardening of the mixture in transfer lines due to cations present in the gellan gum solution.

It has been found that even with the preferred use range of 1.2 - 1.8%, residual material present in the cell paste can cause the gellan gum to set up when the cells are added, depending upon the fermentation medium used to grow the cells. Therefore, to improve control of the immobilization procedure, the cells are preferably washed in deionized water to remove residual salts from the medium, before the cells are combined with the gellan gum. The cell wash step affords better control of the solidification process. That is, the substantially cation-free cell paste can be added to the gellan gum at a lower temperature without premature solidification. Cells can also be washed in other non-ionic solutions of sufficient osmotic strength to prevent cell lysis. Suitable non-ionic solutions include sugar solutions, such as dextrose and sucrose. The cell wash step can be eliminated, but then the amount of cell paste used for a given amount of gellan gum must be reduced. By washing the cells, one may often be able to increase the cell density, and hence density of activity in the immobilized preparation, with a corresponding increase in volumetric productivity of the bioreactor.

Similarly, the procedure is also sensitive to the wetness of the cell paste. Too much water present with the cells will dilute the final concentration of gellan gum below the critical levels needed for acceptable bead formation. The cell paste should be as concentrated as possible, diluted only enough to enable easy transfer and mixing. For purposes of the present invention, the cell content of the cell paste is about 14 wt% based on the dry weight of the cells contained therein. The cell paste should preferably account for less than about 50% of the weight of the gellan gum/cell paste mixture or else the beads will be too soft. A cell paste content of less than 25 wt% is preferred in many cases. If the cell content of the paste is greater than 14%, the cell paste may account for more than 50% of the mixture. The concentration of Mg⁺⁺ ion is not as critical, but at concentrations below 0.1 M there is a noticeable reduction in bead strength.

The immobilized catalytically-active substance prepared according to the above procedure has surprising stability without the additional cross-linking treatments which are normally required for other encapsulation methods. Indeed, in one embodiment it has been determined that the half-life for aspartase activity in E. coli cells (ATCC 11303), encapsulated according to the above procedure, is at least 150 days. This degree of stability is unexpected in light of the fact that the same cells encapsulated in carrageenan without glutaraldehyde treatment only show a 70-90 day half-life, see U.S. Patent 4,526,867. Since the half-life of the free cells is only about 17 days, the immobilization with gellan gum significantly extends the useful time period in which the cells can be utilized for enzymatic reactions.

Activity losses upon immobilization are negligible because of the mild conditions required for encapsulation and hardening. Glutaraldehyde treatment, which may destroy some activity while imparting stability, is not required but may be used without adversely affecting immobilization. Freshly harvested E. coli cells (ATCC 11303) show 7-8 fold lower aspartase activity than the same cells immobilized and activated for 24 hours in substrate at 37°C. The free cells require about 3 days in substrate to reach their peak activity, while immobilized cells are at maximum after 18-24 hours. These differences in timing for peak activity make it difficult to compare activity levels in free cells vs. immobilized cells. Published information on activity retention for other encapsulation methods does not give specifics for timing of assays. The critical timing of activity determinations is shown by the fact that immobilization of free cells at their peak (3 day) activity does not produce an immobilized preparation with as much activity as if fresh cells (with lower activity) were encapsulated.

Those skilled in the art recognize that enzymes which are encapsulated into polymeric substances often have increased Michaelis constants because of the difficulty of diffusing even low molecular weight substrates into the matrix. The Kₘ for soluble aspartase is reported to be 0.15 M (Sato et al., BBA, 570, 179-186, 1979). The Kₘ for gellan gum immobilized E. coli cells (ATCC 11303) containing aspartase was measured experimentally to be 0.2-0.25 M. The fact that the Kₘ for the gellan gum system is close to that of the soluble enzyme is indicative of an immobilization method that does not add hindering environmental effects to the enzyme. This result is entirely unexpected because the Kₘ for aspartase in carrageenan entrapped E. coli cells is 0.71-0.85 M (Sato, 1979). Low apparent Michaelis constant is a desirable and surprising advantage to gellan gum immobilization.

In another embodiment of the present invention, gellan gum can be coated onto ion exchange resin beads and hardened by the cations on the resin. The advantages of this embodiment are: small, mechanically stable beads with thinner transport layers. This is a novel method of cross-linking a gelling agent. Indeed, all prior art methods require a soluble cation to harden the matrix. In this type of embodiment it is preferred that the gellan gum harden within the pores of the resin as well as on the outer surface. This requires that the pores of the resin be large enough to accommodate the microbial cells to be immobilized. If the pores are too small the gellan gum/cell mixture will be forced to the exterior surface of the bead which would, in many cases, not have sufficient cation density to harden the gel.

This invention is equally applicable for a variety of enzyme systems. For example, penicillin acylase, glucose isomerase, glucose oxidase, fumarase, phanylalanine ammonia lyase, aspartate amino transferase, invertase, cis-trans maleic isomerase, and L-aspartate beta-decarboxylase, among others, can all be prepared by the process of this invention. When penicillin acylase is desired, cells of Proteus rettgeri can be employed. When immobilization of cells containing glucose isomerase, glucose oxidase, fumarase, invertase, cis-trans maleic isomerase, or L-aspartate beta-decarboxylase (among others) is desired, microorganisms which may be used can include those of the genera *Streptomyces, Bacillus, Acetobacter, Pseudomonas, and Aspergillus.* Microorganisms of this type may not necessarily be intact living cells, but may be physically or chemically treated prior to use in the present invention.

Those skilled in the art recognize that the present system will be suitable for entrapping partially purified or purified enzymes. Indeed, this approach may be useful in cases where the enzyme of interest is susceptible to attack by proteases. Alternately, in some cases one may desire to remove other enzymes present in the microbial cell which would further catabolize the desired reaction product. In cases where the purified enzymes may leach from the gellan gum bead, one may reduce the loss of enzyme by including a small amount of gelatin and crosslinking with a suitable chemical agent such as glutaraldelhyde.

The following examples are provided to better elucidate the practice of the present invention and are, in no way, intended to limit the scope of the present invention.

### EXAMPLE 1

E. coli cells, ATCC 11303, were grown under standard fermentation conditions in shake flasks. The broth was centrifuged at 8000 x gravity for 8 minutes to give a cell paste -- about 1.2 grams of paste were obtained from 100 ml of broth. The cells were washed in deionized (Milli-Q) water by resuspending 4 gm cells in 5 ml water, centrifuging 5 min at 8000 x gravity, followed by a second wash step. A gellan gum solution was prepared by adding 0.24 gm gellan gum to 19.6 ml water, heating the mixture to 90°C with stirring, and then cooling to 54°C. The washed cells were slurried in 1 ml water and then added to the 54°C gellan gum solution. The gellan gum-cell mixture was immediately pipetted dropwise into a of 0.2 M MgSO₄ solution maintained at room temperature. The beads were gently agitated for 10 minutes to complete hardening and then were vacuum filtered from the MgSO₄ solution. The final weight of the immobilized preparation was 16.9 grams. The immobilized cells were incubated overnight at 37°C in 1.5 M fumaric acid substrate solution. The immobilized cells later showed a rate of 0.0253 moles aspartic produced per gm cells-hour.

### EXAMPLE 2

A highly porous (pore diameter ≧1 micron) cationic exchange resin is put into Mg⁺⁺ion form using standard techniques. The resin is then dried. A 1 wt% solution of gellan gum in deionized water is made by dissolving 0.08 gm of gellan gum in 8 ml of deionized water. The gellan gum solution is heated to about 80°C and then cooled to about 40°C. E. coli cells (ATCC 11303), obtained as described in Example 1, are added to the above-described gellan gum in the amount of about 1 gm of cell paste per 8 ml of gellan gum solution. The gellan gum/cell mixture is coated onto the dry porous cationic exchange resin by hard-stirring the resin with the gellan gum/cell mixture at about 40°C and then permitting the material to cool to room temperature (∼25°C). A sufficient amount of resin is used so that essentially all the material is taken up by the resin. Upon cooling the gellan gum will harden entrapping the cell in and on the resin beads.

### Example 3

Immobilized E. coli cells, ATCC 11303, were prepared as described in Example 1. A column bioreactor was loaded with immobilized cells for half-life determination. The feed stream to the column was 1.5 M NH₄-fumarate (technical grade), 1 mM MgSO₄, at pH 8.5.

During days 1-19 the column was operated 8 hours/day at variable feed rates and included a three-day period when malfunctioning temperature control resulted in approximately 20% loss of enzyme. The bioreactor was operated 24 hours/day, but at variable feed rates from day 20 to day 30. Stability data was obtained from day 31 to 66 at a feed rate of two bed-volumes per hour and 37°C.

The kinetic data indicates a zero time intercept of 67% conversion at two bed-volumes per hour feed rate. Linear regression analysis of the stability data projects a half-life of about 150 days.

For comparison, the half-life of free cells grown in glycerol-fumarate medium, calculated as the time for activity to reach 50% of the peak level, is estimated to be about 17 days. The above data was obtained using batch reactions of 2 hours at 37°C and 1.5 M NH₄-fumarate at pH 8.5.

## Claims

1. A method for immobilization of catalytically-active microbial cells which comprises:
a) admixing a paste of microorganisms with an aqueous solution of gellan gum, having a concentration of gellan gum sufficient to cause formation of a bead upon hardening and not prematurely hardening due to residual material in the microorganism paste; and
b) adding the mixture of part (a) drop-wise to an aqueous solution of cations of sufficient concentration and temperature to promote the hardening of the mixture of part (a) thereby forming a hardened bead containing said microorganisms; and
c) recovering the hardened bead produced in step (b).

2. A method of Claim 1 in which the aqueous gellan gum solution has a concentration of gellan gum between 1.2 and 1.8 wt% and the microorganism paste comprises less than about 50 wt% of the resulting mixture.

3. A method of Claim 2 in which the microorganism possesses aspartase activity.

4. A method of Claim 2 in which the microorganism is E. coli.

5. A method of Claim 2 in which the microorganism is E. coli (ATCC 11303).

6. A method of Claim 2 in which the aqueous cationic solution comprises magnesium ions at a concentration between about 0.1 and 0.4 M.

7. A method of Claim 2 in which the gellan gum has a concentration between 1.2 and 1.6 wt%.

8. A method for immobilization of catalytically-active microbial cells which comprises:
a) admixing a paste of microorganisms with an aqueous solution of gellan gum having a concentration of gellan gum between about 1.2 and 1.8 wt%, said microorganism paste comprising less than about 50 wt% of the resulting mixture; and
b) admixing the mixture of part (a) with a porous cationic exchange resin, said resin being porous enough to permit ingress of microorganisms and having a sufficient cationic charge density to permit hardening of the gellan gum.

9. A method of Claim 8 in which the microorganism possesses aspartase activity.

10. A method of Claim 8 in which the microorganism is E. coli.

11. A method of Claim 8 in which the microorganism is E. coli (ATCC 11303).

12. A method of Claim 8 in which the cationic exchange resin is in magnesium ion form.

## Patentansprüche

1. Verfahren zur Immobilisierung katalytisch aktiver Mikrobenzellen, umfassend:
a) Vermischen einer Mikroorganismenpaste mit einer wäßrigen Gellangummilösung mit einer ausreichenden Gellangummikonzentration, um bei der Härtung die Bildung eines Kügelchens zu bewirken und nicht vorzeitig aufgrund von restlichem Material in der Mikroorganismenpaste zu härten; und
b) tropfenweises Zugeben der Mischung aus Teil (a) zu einer wäßrigen Kationenlösung einer ausreichenden Konzentration und Temperatur, um das Härten der Mischung aus Teil (a) zu fördern, wodurch ein die Mikroorganismen enthaltendes, gehärtetes Kügelchen gebildet wird; und
c) Gewinnen des in Stufe (b) hergestellten gehärteten Kügelchens.

2. Verfahren nach Anspruch 1, wobei die wäßrige Gellangummilösung eine Gellangummikonzentration zwischen 1,2 und 1,8 Gew.% besitzt und die Mikroorganismenpaste weniger als etwa 50 Gew.% der resultierenden Mischung umfaßt.

3. Verfahren nach Anspruch 2, wobei der Mikroorganismus Aspartaseaktivität besitzt.

4. Verfahren nach Anspruch 2, wobei der Mikroorganismus E. coli ist.

5. Verfahren nach Anspruch 2, wobei der Mikroorganismus E. coli (ATCC 11303) ist.

6. Verfahren nach Anspruch 2, wobei die wäßrige Kationenlösung Magnesiumionen in einer Konzentration zwischen etwa 0,1 und 0,4 M umfaßt.

7. Verfahren nach Anspruch 2, wobei die Gellangummikonzentration zwischen 1,2 und 1,6 Gew.% liegt.

8. Verfahren zur Immobilisierung katalytisch aktiver Mikrobenzellen, umfassend
a) Vermischen einer Mikroorganismenpaste mit einer wäßrigen Gellangummilösung mit einer Gellangummikonzentration zwischen etwa 1,2 und 1,8 Gew.%, wobei die Mikroorganismenpaste weniger als etwa 50 Gew.% der resultierenden Mischung umfaßt; und
b) Vermischen der Mischung aus Teil (a) mit einem porösen Kationenaustauscherharz, wobei das Harz ausreichend porös ist, um ein Eindringen der Mikroorganismen zu ermöglichen und eine ausreichende Kationenladungsdichte besitzt, um eine Härtung des Gellangummis zu ermöglichen.

9. Verfahren nach Anspruch 8, wobei der Mikroorganismus Aspartaseaktivität besitzt.

10. Verfahren nach Anspruch 8, wobei der Mikroorganismus E. coli ist.

11. Verfahren nach Anspruch 8, wobei der Mikroorganismus E. coli (ATCC 11303) ist.

12. Verfahren nach Anspruch 8, wobei das Kationenaustauscherharz in der Magnesiumionenform vorliegt.

## Revendications

1. Procédé d'immobilisation de cellules microbiennes catalytiquement actives qui comprend :
a) le mélange d'une pâte de microorganisme avec une solution aqueuse de gomme "gellan" ayant une concentration suffisante en gomme "gellan" pour provoquer par durcissement la formation d'une goutte ou perle et de ne pas durcir prématurément du fait de matériaux résiduels dans la pâte de microorganisme; et
b) l'addition, goutte-à-goutte, du mélange de la partie (a) à une solution aqueuse de cations ayant une concentration et une température suffisantes pour promouvoir le durcissement du mélange de la partie (a) formant ainsi une goutte ou perle durcie contenant lesdits microorganismes; et
c) récupérer la goutte ou perle durcie produite à l'étape (b).

2. Procédé selon la revendication 1, où la solution aqueuse de gomme "gellan" a une concentration en gomme "gellan" comprise entre 1, 2 et 1, 8 % en poids et la pâte de microorganisme constitue moins d'environ 50 % du mélange résultant.

3. Procédé selon la revendication 2, où les microorganismes possèdent une activité d'aspartase.

4. Procédé selon la revendication 2, où le microorganisme est E. Coli.

5. Procédé selon la revendication 2, où le microorganisme est E. Coli (ATCC 11303).

6. Procédé selon la revendication 2, où la solution aqueuse cationique comprend des ions magnésium à une concentration comprise entre environ 0,1 et 0,4 M.

7. Procédé selon la revendication 2, où la gomme "gellan" a une concentration comprise entre 1, 2 et 1, 6 % en poids.

8. Procédé d'immobilisation de cellules microbiennes catalytiquement actives, qui comprend :
a) le mélange d'une pâte de microorganisme avec une solution aqueuse de gomme "gellan" ayant une concentration en gomme "gellan" comprise entre environ 1, 2 et 1, 8 % en poids, cette pâte de microorganisme constituant moins d'environ 50 % en poids du mélange résultant; et
b) mélanger le mélange résultant de l'étape (a) avec une résine poreuse échangeuse de cations, cette résine étant suffisamment poreuse pour permettre l'entrée des microorganismes et ayant une densité de charge cationique suffisante pour permettre le durcissement de la gomme "gellan".

9. Procédé selon la revendication 8, où le microorganisme possède une activité d'aspartase.

10. Procédé selon la revendication 8, où le microorganisme est E. Coli

11. Procédé selon la revendication 8, où le microorganisme est E. Coli (ATCC 11303).

12. Procédé selon la revendication 8, où la résine échangeuse cationique est sous forme d'ion magnésium.
